# EUROPEAN PATENT SPECIFICATION

(11) **EP 1 477 157 B1**
(45) Date of publication and mention of the grant of the patent: **19.03.2008**
(21) Application number: 04252687.1
(22) Date of filing: 07.05.2004
(51) Int. Cl.: A61K 8/34, A61K 8/41, A61K 8/42, A61Q 9/04

(54) **Compositions comprising an amidine and an alkane polyol**
Zusammensetzungen enthaltend Amidine und Alkan Polyol
Compositions conprenant une amidine et un alkane polyol

(30) Priority: 16.05.2003 EP 03253081
(43) Date of publication of application: 17.11.2004
(73) Proprietor: THE PROCTER & GAMBLE COMPANY, Cincinnati, Ohio 45202 (US)
(72) Inventor: Elliott, Russel Philip, Egham Surrey TW20 9RJ (GB); McKay, Barnaby George Robert, Muswell Hill London N10 1HJ (GB); VanOosthuyze, Kristina Emma Inge, Horsell Woking Surrey GU21 4XD (GB)
(74) Representative: Wilding, Richard Alan

(56) References cited:
- WO-A-88/08295
- US-B1- 6 184 252

## Description

### TECHNICAL FIELD

The present invention relates to compositions comprising an amidine, an alkane polyol having a ClogP of from about +0.2 to about +1.55 and water. The compositions of the present invention maintain the physical and chemical stability of the amidine in aqueous solutions, generating compositions that are useful for hair growth inhibition having good application characteristics.

### BACKGROUND OF INVENTION

A main function of mammalian hair growth is to provide environmental protection. However, that function has been lost in humans, in whom hair is kept or removed from various parts of the body, essentially for cosmetic reasons. For example, for women in the United States, it is generally preferred to have hair on the scalp, but not on the legs, underarms, or certain areas of the face.

Various procedures and personal care products have been developed to remove unwanted hair, including shaving, electrolysis, depilatory creams or lotions, depilatory devices, waxing, plucking, therapeutic androgens, and laser hair removal. However, such conventional procedures frequently have drawbacks associated with them. Shaving, for instance, may cause nicks, cuts, rash and irritation and often leave undesirable stubble, as well as having to be carried out frequently. Electrolysis and laser hair removal can keep a treated area free of hair for prolonged periods of time, but can be either expensive, painful, and/or sometimes leave scarring. Waxing and plucking are painful and are poor options for shorter hair. Anti-androgens, used to treat female hirsutism, can have unpleasant physical side effects as well as possible birth defect implications. Since in non-hirsutistic females, the role of androgens is not required for normal hair growth, there is a need for technologies that do not function via androgen mediated pathways due to the birth defect implications. Depilatory devices can be painful to use and are not well-suited for many areas of the body including underarms and face. Finally, depilatory creams, although effective, are messy to apply and typically are not recommended for frequent use due to their high irritancy potential.

It has recently been discovered that amidine compounds are useful for regulating mammalian hair growth, including retarding, inhibiting, or eliminating hair growth (see copending US Patent Application serial number US60/451910). However, these materials are physically and, to a lesser extent, chemically unstable when in aqueous solution, and are prone to crystalline precipitation. Aqueous compositions comprising amidines are difficult to formulate at low levels, especially greater than 0.01% without the amidines crystallising out of solution either in the product during storage, or on the skin once applied. Surprisingly it has been found that this aqueous instability can be overcome by incorporating these materials in compositions in combination with specific alkane polyols having a select ClogP value.

### SUMMARY

The present invention relates to a composition comprising:
a) an amidine:
b) an alkane polyol having an ClogP value of from +0.2 to +1.55; and
c) water.

Furthermore, the present invention relates to methods of inhibiting mammalian hair growth, the use of alkane polyols having a ClogP of from +0.2 to +1.55 for modulating the delivery and efficacy of hair growth inhibitors on the skin, use of alkane polyols of the present invention as hair growth inhibitors, and to methods of manufacture of the compositions of the present invention.

These and other features, aspects, and advantages of the present invention will become evident to those skilled in the art from a reading of the present disclosure.

### DETAILED DESCRIPTION

While the specification concludes with the claims particularly pointing and distinctly claiming the invention, it is believed that the present invention will be better understood from the following description.

All percentages, parts and ratios are based upon the total weight of the cosmetic compositions of the present invention and all measurements made are at 25°C, unless otherwise specified. All such weights as they pertain to listed ingredients are based on the active level and, therefore, do not include carriers or by-products that may be included in commercially available materials, unless otherwise specified.

The compositions of the present invention are useful for regulating the growth of mammalian hair. The desire for hair growth regulation may also stem from hair growth patterns induced or caused by factors internal and/or external to the body.

"Regulating hair growth," namely mammalian hair growth, includes reducing, modulating, inhibiting, attenuating, retarding, promoting, enhancing, and/or the diminuation of hair growth.

"Reduction/Inhibition of hair growth," as used herein, is demonstrated when the frequency of hair removal is reduced, or the tactile and visual feel of mammalian hair is improved.

"Mammalian hair," as referenced herein, includes hair on any part of the body of a mammal and may include facial, cranial, or body hair. The compositions of the present invention are particularly suitable for reducing the growth of unwanted hair in women suffering from hirsutism or other conditions.

The term "improving the tactile and visual feel" as used herein, refers to the more noticeable improvement in the appearance of the hair on the skin such that it is perceived to be softer, finer, less noticeable. Additionally, the ease, frequency, and effectiveness of shaving will be perceived by the mammal. The mammal shall perceive reduced frequency in shaving.

The term "keratinous tissue," as used herein, refers to keratin-containing layers disposed as the outermost protective covering of mammals (e.g., humans, dogs, cats, etc.) which includes, but is not limited to, skin, hair, etc.

The term "topical application", as used herein, means to apply or spread the compositions of the present invention onto the surface of the keratinous tissue.

The term "dermatologically-acceptable," as used herein, means that the compositions or components thereof so described are suitable for use in contact with mammalian keratinous tissue without undue toxicity, incompatibility, instability, allergic response, and the like.

The term "safe and effective amount" as used herein means an amount of a compound or composition sufficient to significantly induce a positive benefit, preferably a hair growth regulating benefit, or positive hair appearance or feel benefit, including independently or in combinations the benefits disclosed herein, but low enough to avoid serious side effects, i.e., to provide a reasonable benefit to risk ratio, within the scope of sound judgment of the skilled artisan.

The compositions of the present invention, including the essential and optional components thereof, are described in detail hereinafter.

The compositions of the present invention comprise an amidine. Amidines are useful in the compositions of the present invention as hair growth inhibitors. As used herein "amidine" includes materials conforming to the general formula I: 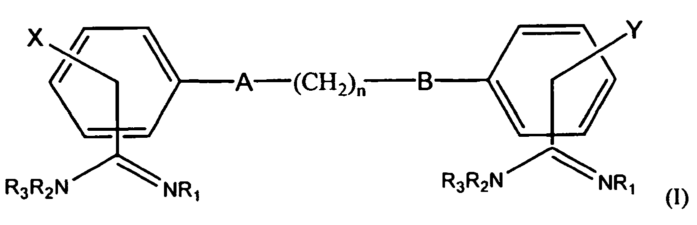 wherein A and B are independently selected from O, N, or nil, n = 3 to 10, X and Y are independently selected from H, nitro, amino, hydroxyl, sulfonate, or C₁-C₃ alkyl, and R₁, R₂ and R₃ are independently selected from H, C₁-C₄ alkyl, C₁-C₄ alcohol, formate, acetate or propionate. Preferably, the compositions of the present invention comprise a hair growth inhibitor conforming to formula I wherein A and B are O, N or nil, n = 5 to 8, X and Y are H, and R₁, R₂, and R₃ are independently selected from H, C₁-C₄ alkyl or C₁ to C₄ alcohol, more preferably, where A and B are O, n = 5 or 6, X and Y are H, and R₁, R₂ and R₃ are H.

As used herein, "amidine" also includes materials conforming to the general formula II: 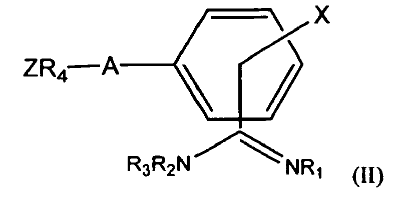 wherein A is selected from O, N, or nil, R₁, R₂ and R₃ are independently selected from H, C₁-C₄ alkyl, C₁-C₄ alcohol, formate, acetate or propionate, X is selected from H, nitro, amino, hydroxyl, sulfonate, or C₁-C₃ alkyl, R₄ is selected from linear or branched, saturated or unsaturated C₃-C₂₀ hydrocarbon, or nil; and Z is H, OH or NH₂.

Non-limiting examples of amidine hair growth inhibitors useful in the present invention include:

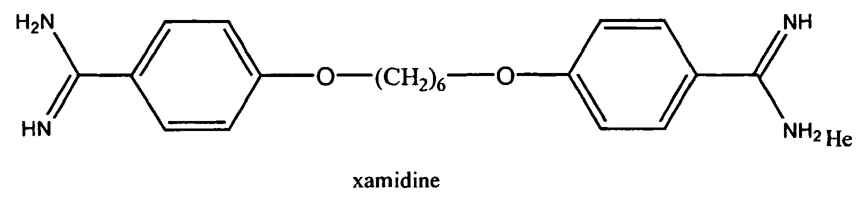

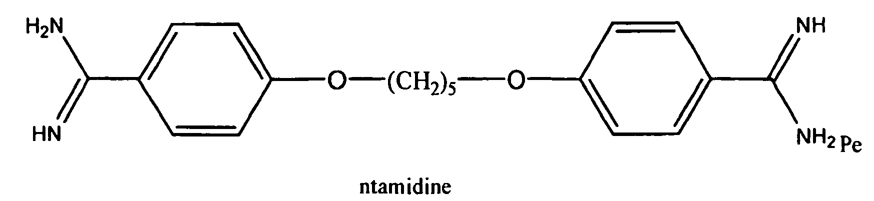

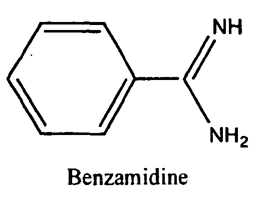

More preferably still, the compositions of the present invention comprise a safe and effective amount of hexamidine, its salts, and derivatives. More preferably, the hexamidine is hexamidine isethionate. As used herein, "hexamidine" includes any isomers and tautomers of such and is commercially available as hexamidine isethionate under the tradename Elastab™ HP100 from Laboratoires Serobiologiques (Pulnoy, France).

The compositions of the present invention preferably comprise from about 0.001% to about 10% of the amidine, more preferably from about 0.01% to about 5%, and more preferably still from about 0.05% to about 2.0% by weight of the composition.

The compositions of the present invention also comprise an alkane polyol having an N-octanol/water partition coefficient (ClogP) of from +0.2 to +1.55. It has surprisingly been found that alkane polyols having a ClogP of from +0.2 to +1.55 are able to stabilise the amidines described herein in aqueous solution. Without wishing to be bound by theory, it is believed that the alkane polyols of the present invention are able to stabilise the amidine by forming transient micelle structures with the amidine, thereby stabilising them in solution, and preventing their interaction and crystal formation. The ClogP values of these materials indicate their hydrophilic/lipophilic balance. Alkane polyols having a ClogP value of between +0.2 and +1.55 are slightly lipophilic, enabling them to interact with the amidines. However, the alkane polyols herein maintain a high enough solubility in water due to the presence of the hydroxy moieties to allow them to maintain the amidine in solution. These materials do not form true micells with the amidine, such as those formed by surfactants, as they still allow the amidine to be deposited onto the skin when the compositions are topically applied.

It has also been found that the alkane polyols herein are able to maintain the stability of the aqueous formulation once the compositions of the present invention are topically applied. This is desirable because, once topically applied, the compositions lose water via evaporation, resulting in a progressively higher concentration of the amidines of the present invention, and other non-volatile components such as salts and thickeners. Without the alkane polyols of the present invention, this increase in concentration results in the amidines forming crystals on the skin, generating a solid, crystalline phase on the skin that is undesirable to consumers and having limited efficacy. It has been found that incorporation of the alkane polyols herein improves the topical application characteristics of the compositions herein, as well as improving their stability.

Furthermore, it has surprisingly been found that incorporation of the alkane polyols described herein in cosmetic compositions increase synergistically the efficacy of the hair growth inhibitors. Without wishing to be bound by theory, it is believed that this may be due in part to the hair growth inhibition activity of the alkane polyols themselves, and/or the ability of the alkane polyols to potentiate the delivery of amidines to the hair follicle via either transdermal penetration or a shunt route.

As used herein, the term "ClogP" means the logarithm to base 10 of the octanol/water partition coefficient (P). The octanol/water partition coefficient of a material is the ratio between its equilibrium concentrations in octanol and water. Given that this measure is a ratio of the equilibrium concentration of a material in a non-polar solvent (octanol) with its concentration in a polar solvent (water), ClogP is also a measure of the hydrophobicity of a material - the higher the ClogP value, the more hydrophobic the material. The ClogP values herein are calculated using the Advanced Chemistry Development (ACD) Solaris software Version 4.67, utilising the display property format on the CAS Online service via STN of Cleveland Ohio. The ClogP values are determined by the fragment approach. The fragment approach is based on the chemical structure of a compound and takes into account the numbers and type of atoms, the atom connectivity, and chemical bonding. The ClogP values, which are the most reliable and widely used estimates for this physiochemical property, are used herein.

The ClogP of the alkane polyols of the present invention is from +0.2 to +1.55, preferably from +0.2 to +1.35, more preferably from +0.2 to +1.0.

Preferred alkane polyols for use herein comprise C₆ to C₉ alkane polyols, or mixtures thereof, more preferably C₆ to C₉ alkane diols, or mixtures thereof. Non-limiting examples of alkane polyols suitable for use herein include 1,2-hexanediol, 2-ethyl-1,3-hexanediol, 1,2-heptanediol, 1,7-heptanediol, 1,2-octanediol, 1,8-octanediol, 1,9-nonanediol, or mixtures thereof. Table 1 summarises the ClogP values of these materials, and other alkane polyols.

**Table 1.**

| **Alkane Polyol** | **ClogP** | **Stabilises Amidines?** |
|---|---|---|
| Glycerin | -2.32 | No |
| Propylene glycol | -1.34 | No |
| 2,5-hexanediol | -0.33 | No |
| 1,2 Pentanediol | -0.28 | No |
| 1,6-hexanediol | -0.07 | No |
| Hexylene glycol | 0.0 | No |
| 1,2-hexanediol | +0.25 | Yes |
| 1,7-heptanediol | +0.46 | Yes |
| 1,2-heptanediol | +0.78 | Yes |
| 1,8-octanediol | +0.99 | Yes |
| 2-ethyl-1,3-hexanediol | +1.25 | Yes |
| 1,2-octanediol | +1.32 | Yes |
| 1,9-nonanediol | +1.53 | Yes |
| 1,10-decanediol | +2.06 | No |
| 1,2-decanediol | +2.38 | No |
| Phytantriol | +5.79 | No |

More preferably still, the alkane polyol herein comprises C₆-C₈ alkane diols or mixtures thereof, non-limiting examples of which include 1,2-hexanediol, 2-ethyl-1,3-hexanediol, 1,2-heptanediol, 1,7-heptanediol, 1,2-octanediol, 1,8-octanediol, or mixtures thereof, preferably 1,2-hexanediol.

The compositions of the present invention preferably comprise from 0.01% to 20% of an alkane polyol having a ClogP of from +0.2 to +1.55, more preferably from 0.05% to 10%, more preferably still from 0.5% to 3%, by weight of the composition.

The compositions of the present invention also comprise water. The compositions of the present invention preferably comprise at least 20% water, more preferably at least 30% water, more preferably still at least 40% water.

The compositions of the present invention may further comprise an additional skin care active. Additional skin care actives suitable for use herein include BHT or BHA, cetyl pyridinium chloride, green tea catechins, phytosterols, their salts and derivatives, natural anti-inflammatory agents, antimicrobials, antifungal agents, sunscreens, vitamin B₃ compounds, panthenol or its derivatives, and mixtures thereof. Preferably, the compositions of the present invention comprise an additional skin care active comprising BHT or BHA, vitamin B₃ compounds, panthenol or its derivatives, or mixtures thereof, preferably BHT.

One class of skin care actives suitable for use herein includes BHT or BHA. The BHTs useful herein can be described by the general structure: 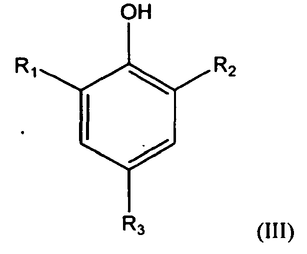 where R₁, R₂ and R₃ are independently selected from H, C₁-C₅ linear or branched alkyl, or mixtures thereof, preferably where R₁ and R₂ are *tert-*butyl and R₃ is methyl.

In the compositions of the present invention, BHT or BHA preferably comprises from about 0.01% to about 2%, and more preferably from about 0.1% to about 1.0%.

A further class of additional skin care actives suitable for use herein includes cetyl pyridinium chloride. Alternate forms of cetyl pyridinium chloride include those in which one or two of the substitutes on the quaternary nitrogen have a carbon chain length (typically alkyl group) typically from about 10 to about 18 carbon atoms. When present, cetyl pyridinium chloride preferably comprises from about 0.001% to about 2%, more preferably from about 0.01% to about 1%, and more preferably still from about 0.05% to about 0.5%.

Another class of additional skin care actives suitable for use herein includes the catechins. Catechin compounds useful herein include catechin, epicatechin, epigallocatechin, epicatechin gallate, epigallocatechin gallate, gallocatechin, and mixtures thereof. More preferably, the catechin is free of caffeine and is extracted and enriched for from a green tea plant source. More preferably still, the catechin is epigallocatechin gallate.

In the composition of the present invention, the catechin mixture preferably comprises from about 0.01% to about 5%, and more preferably from about 0.1% to about 2.5%, by weight of the composition.

A further class of additional skin care active suitable for use herein includes the phytosterols. Phytosterols suitable herein include β-sitosterol, campesterol, brassicasterol, Δ5-avennasterol, lupenol, α-spinasterol, stigmasterol, their derivatives, and mixtures thereof. Non-limiting examples of phytosterols useful herein include β-sitosterol, campesterol, brassicasterol, stigmasterol, and mixtures thereof. More preferably, the phytosterol comprises stigmasterol.

Phytosterols of the present invention can be synthetic or natural in origin and can be used as essentially pure compounds or mixtures of compounds (e.g., extracts from natural sources). Phytosterols are generally found in the unsaponifiable portion of vegetable oils and fats and are available as free sterols, acetylated derivatives, sterol esters, ethoxylated or glycosidic derivatives. More preferably, the phytosterols are free sterols. As used herein, "phytosterol" includes isomers, tautomers, and derivatives (e.g., esters) of such and are commercially available from Aldrich Chemical Company (Milwaukee, Wisconsin), Sigma Chemical Company (St. Louis, Missouri), Cognis, and Karlshamns (Karlshamns, Sweden).

In the compositions of the present invention, the phytosterol preferably comprises from about 0.2% to about 10%, more preferably from about 0.5% to about 10%.

Another class of additional skin care active suitable for use herein comprises a vitamin B₃ compound. As used herein, "vitamin B₃ compound" includes compounds having the formula: 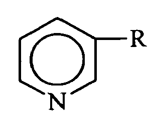 wherein R is - CONH₂ (i.e., niacinamide), - COOH (i.e., nicotinic acid) or - CH₂OH (i.e., nicotinyl alcohol); derivatives thereof; and salts of any of the foregoing. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye). Non-vasodilating esters of nicotinic acid include tocopherol nicotinate and inositol hexanicotinate; tocopherol nicotinate is preferred. The vitamin B₃ compound is preferably used in an amount of from about 0.1% to about 10%, more preferably from about 2% to about 5%.

Preferably, the compositions of the present invention may further comprise panthenol or its derivatives. Panthenol or its derivatives are desirable for use herein, as these materials are able to both regulate and improve the condition of the skin, and also act as hair growth inhibitors. It has been found that the combination of panthenol or its derivatives with the alkane polyols defined in the present invention results in a synergistic increase in the levels of hair growth inhibition when compared with panthenol, or its derivatives alone. Without wishing to be bound by theory, it is believed that this is due to two effects. Firstly, it is believed that the alkane polyols may act as hair growth inhibitors themselves. Secondly, the alkane polyols may also provide a suitable vehicle for the efficient delivery of panthenol, and other hair growth inhibiting compounds including amdines to the hair follicle. It is believed that this is due to the lipophilic nature of the hydrocarbon chain of the alkane polyols allowing it to penetrate into the hair follicle and the cells surrounding it, and consequently delivering the hair growth inhibitor to the hair follicle with greater efficiency, and therefore at greater levels, resulting in increased hair growth inhibition.

The panthenol and its derivatives include D-panthenol ([R]-2,4-dihydroxy-N-[3-hydroxy-propyl)]-3,3-dimethylbutamide), DL-panthenol, pantothenic acids and their salts, preferably the calcium salt, panthenyl triacetate, royal jelly, panthetine, pantotheine, panthenyl ethyl ether, pangamic acid, pantoyl lactose, Vitamin B complex, or mixtures thereof. The compositions of this invention may comprise a safe and effective amount of the panthenol or its derivative, such that the resultant composition is safe and effective for regulating skin texture without excessive stickiness. The panthenol derivative is preferably used in an amount of from about 0.01% to 10%, more preferably from 0.1% to about 5%, more preferably still from about 0.2% to about 3%.

A further class of additional skin care actives useful herein comprises retinoids. As used herein, "retinoid" includes all natural and/or synthetic analogs of Vitamin A or retinal-like compounds which possess the biological activity of Vitamin A in the skin as well as the geometric isomers and stereoisomers of these compounds. The retinoid is preferably retinal, including retinol esters (e.g., C₂ - C₂₂ alkyl esters of retinol, including retinyl palmitate, retinyl acetate, retinyl proprionate), retinal, and/or retinoic acid (including all-trans retinoic acid and/or 13-cis-retinoic acid), more preferably retinoids other than retinoic acid. These compounds are well known in the art and are commercially available from a number of sources, e.g., Sigma Chemical Company (St. Louis, MO), and Boerhinger Mannheim (Indianapolis, IN). Other retinoids which are useful herein are described in U.S. Patent Nos. 4,677,120; 4,885,311; 5,049,584; 5,124,356; and Reissue 34,075. Other suitable retinoids are tocopheryl-retinoate [tocopherol ester of retinoic acid (trans- or cis-), adapalene {6-[3-(1-adamantyl)-4-methoxyphenyl]-2-naphthoic acid}, and tazarotene (ethyl 6-[2-(4,4-dimethylthiochroman-6-yl)-ethynyl]nicotinate). One or more retinoids may be used herein. Preferred retinoids are retinol, retinyl palmitate, retinyl acetate, retinyl proprionate, retinal and combinations thereof. More preferred are retinol and retinyl palmitate.

The compositions of this invention may contain a safe and effective amount of the retinoid, such that the resultant composition is safe and effective for regulating skin texture. The compositions preferably contain from about 0.005% to about 2%, more preferably 0.01 % to about 2%, retinoid. Retinol is preferably used in an amount of from or about 0.01% to about 0.15%; retinol esters are most preferably used in an amount of from or about 0.01 % to about 2%; retinoic acids are most preferably used in an amount of from about 0.01% to about 0.25%; tocopheryl-retinoate [tocopherol ester of retinoic acid (trans- or cis), adapalene {6-[3-(1-adamantyl)-4-methoxyphenyl]-2-naphthoic acid}, and tazarotene are more preferably used in an amount of from about 0.01% to about 2%.

Another additional skin care active suitable for use herein includes the so-called "natural" anti-inflammatory agents. Such agents may suitably be obtained as an extract by suitable physical and/or chemical isolation from natural sources (e.g., plants, fungi, by-products of microorganisms). For example, candelilla wax, alpha-bisabolol, aloe vera, Manjistha (extracted from plants in the genus Rubia, particularly Rubia Cordifolia), and Guggal (extracted from plants in the genus Commiphora, particularly Commiphora Mukul), kola extract, chamomile, red clover extract, and sea whip extract, may be used.

Additional natural anti-inflammatory agents useful herein include allantoin and compounds of the Licorice (the plant genus/species Glycyrrhiza glabra) family, including glycyrrhetic acid, glycyrrhizic acid, and derivatives (e.g., salts and esters). Specific examples of the foregoing include oil soluble licorice extract, the glycyrrhizic and glycyrrhetic acids themselves, monoammonium glycyrrhizinate, monopotassium glycyrrhizinate, dipotassium glycyrrhizinate, 1-beta-glycyrrhetic acid, stearyl glycyrrhetinate, and 3-stearyloxy-glycyrrhetinic acid, and disodium 3-succinyloxy-beta-glycyrrhetinate. Stearyl glycyrrhetinate is preferred.

The active component of these anti-inflammatory agents (e.g., biabolol, glycyrrhetinate esters) may also be obtained via extraction from natural sources or prepared synthetically. When present, the compositions of the present invention preferably comprise from 0.001% to 5%, more preferably from 0.001% to 1% of the natural anti-inflammatory agents.

The compositions of the present invention may comprise an antimicrobial or antifungal active. Such actives are capable of destroying microbes, preventing the development of microbes or preventing the pathogenic action of microbes. A safe and effective amount of an antimicrobial or antifungal active may be added to the present compositions, preferably, from about 0.001% to about 10%, more preferably from about 0.01% to about 5%, and most preferably from about 0.05% to about 2%.

Generally, the sunscreens can comprise from about 0.5% to about 20% of the compositions useful herein. Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, August 25, 1978.

Also particularly useful in the compositions are sunscreen actives such as those disclosed in U.S. Patent No. 4,937,370, and U.S. Patent No. 4,999,186. The sunscreening agents disclosed therein have, in a single molecule, two distinct chromophore moieties, which exhibit different ultra-violet radiation absorption spectra. One of the chromophore moieties absorbs predominantly in the UVB radiation range and the other absorbs strongly in the UVA radiation range.

Preferably, the compositions of the present invention further comprise humectants. Humectants are desirable in the compositions of the present invention as they impart water-binding capacity to the skin, increasing the acute and chronic hydration status of the skin, whilst also repairing the skin barrier. This results in the skin feeling and looking moisturised and healthy. The compositions of the present invention may comprise from 0.01% to 30%, preferably from 0.1% to 20%, more preferably still from 0.5% to 15% of humectant by weight of the composition. Suitable humectants useful herein include polyhydric alcohols, sodium 2-pyrrolidone-5-carboxylate (NaPCA), amino acids and derivatives, guanidine; glycolic acid and glycolate salts (e.g. ammonium and quaternary alkyl ammonium); lactic acid and lactate salts (e.g. ammonium and quaternary alkyl ammonium); other alpha hydroxy acids such as malic acid, aloe vera in any of its variety of forms (e.g., aloe vera gel); hyaluronic acid, precursors and derivatives thereof (e.g., glucosamine and salt derivatives such as sodium hyaluronate); lactamide monoethanolamine; acetamide monoethanolamine; urea; and mixtures thereof. Preferred for use in the compositions of the present invention are polyhydric alcohols.

The polyhydric alcohol humectants herein are not suitable for use as an alkane polyol having a ClogP of from +0.2 to +1.55. The polyhydric alcohol humectants herein typically have a ClogP of less than 0.05. Suitable polyhydric alcohols for use herein as humectants include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexane triol (e.g., 1,2,6-hexanetriol), trimethylol propane, neopentyl glycol, glycerine, ethoxylated glycerine and propoxylated glycerine. Preferred polyhydric alcohols of the present invention are polyhydric alcohols with 3 to 9 carbon atoms in the molecule. Examples include glycerine, butylene glycol, propylene glycol, dipropylene glycol, polyethylene glycol and derivatives thereof, hexane triol, ethoxylated glycerine and propoxylated glycerine, and mixtures thereof. More preferred for use in the present invention is glycerin. The compositions of the present invention comprise from about 0.01% to about 30% polyhydric alcohol, preferably from about 0.1% to about 20% by weight of the composition.

A further class of humectants includes the amino acids and their derivatives. Suitable amino acids for use herein include both D- and L- isomers of naturally occurring amino acids. Suitable examples include L-isomers of serine, alanine, proline and hydroxyproline.

The compositions of the present invention may be formulated in any physical form known to those skilled in the art. Preferably the compositions of the present invention are formulated as cosmetic compositions, more preferably as hair growth inhibition compositions. Suitable cosmetic formulations useful herein include, but are not limited to, toilet bars, liquids, shampoos, gels, clear lotions, bath gels, hair conditioners, hair tonics, pastes, mousses, foundations, lipsticks, rouges, mascaras, deodorants, skin creams, skin lotions, aerosols, spray gels and the like. Preferably, the cosmetic compositions of the present invention are in the form of skin creams, skin lotions, deodorants or spray gels.

The compositions of the present invention are preferably formulated to have a pH that is acceptable for application to mammalian skin and hair. Preferably the pH of the compositions of the present invention is from 4 to 7.5. It has been found that the amidines of the present invention are unstable in solution at a pH above 7.5 due to hydrolysis of the amidine. More preferably, the cosmetic compositions of the present invention have a pH of from 5 to 6.5.

The compositions herein are preferably in the form of a water-in-oil or oil-in-water emulsion. Preferably the cosmetic compositions herein are oil-in-water emulsions wherein the composition comprises one or more aqueous phases in an oily continuous phase. The total level of oil phase components in the compositions of the invention is typically from about 0.1 % to about 60%, preferably from about 1% to about 30%, more preferably from about 2% to about 20% and most preferably from about 3% to about 10%. Preferably, the compositions of the present invention comprise a water-in-silicone emulsion. Water-in-silicone emulsions contain a continuous silicone phase and a dispersed aqueous phase. Preferred water-in-silicone emulsions of the present invention comprise from about 1% to about 60%, preferably from about 5% to about 40%, more preferably from about 10% to about 20%, by weight of a continuous silicone phase. The continuous silicone phase exists as an external phase that contains or surrounds the discontinuous aqueous phase. Water-in-silicone emulsions of this type are described in copending U.S. Patent Application Serial No. 08/570,275, filed December 11, 1995, in the names of Joseph Michael Zukowski, Brent William Mason, Larry Richard Robinson and Greg George Hillebrand.

The oil phase preferably comprises oily components such as a natural or synthetic oils selected from mineral, vegetable, and animal oils, fats and waxes, fatty acid esters, fatty alcohols, fatty acids and mixtures thereof. Non-limiting examples for use herein include saturated and unsaturated fatty alcohols such as behenyl alcohol, cetyl alcohol and stearyl alcohol and hydrocarbons such as mineral oils or petrolatum.

The present compositions preferably comprise a silicone phase. The silicone phase can comprise one or more silicone components such as silicone fluids, gums, and mixtures thereof. The, or each, silicone phase generally comprises from about 0.1% to about 20%, preferably from about 0.2% to about 10%, more preferably from about 0.3% to about 5%, of the composition.

Silicone components can be fluids, including straight chain, branched and cyclic silicones. Suitable silicone fluids useful herein include polyalkyl siloxane fluids, polyaryl siloxane fluids, cyclic and linear polyalkylsiloxanes, and mixtures thereof. The silicone fluids can be volatile or non-volatile. Suitable examples include polydimethyl siloxanes available, for example, from the General Electric Company as the SF and Viscasil™ series and from Dow Coming as the Dow Coming 200 series. Also useful in the compositions of the present invention are silicone elastomer materials such as DC9040™ available from Dow Corning. Also useful are essentially non-volatile polyalkylarylsiloxanes, for example, polymethylphenylsiloxanes, having viscosities of about 0.65 to 30,000 mm².s⁻¹ at 25°C. These siloxanes are available, for example, from the General Electric Company as SF 1075 methyl phenyl fluid or from Dow Coming as 556 Cosmetic Grade Fluid. Also useful herein are polyether siloxane copolymers such as polydiorganosiloxane-polyoxyalkylene copolymers containing at least one polydiorganosiloxane segment and at least one polyoxyalkylene segment. The silicone components can also comprise silicone gums. including non-volatile polyalkyl and polyaryl siloxane gums. In preferred embodiments, a silicone oil phase comprises a silicone gum or a mixture of silicones including the silicone gum.

Useful herein are silicone/gum fluid blends. Preferred silicone-gum fluid blend based component for use in the compositions herein is a dimethiconol gum having a molecular weight of from about 200,000 to about 4,000,000 along with a silicone fluid carrier with a viscosity of about 0.65 to 100 mm².s⁻¹. An example of this silicone component is Dow Coming Q2-1503 (85% 5 mm².s⁻¹ Dimethicone Fluid/15% Dimethiconol gum) and Dow Coming Q2-1501, both available from Dow Coming.

The compositions of the present invention preferably comprise emollient materials including branched chain hydrocarbons having an weight average molecular weight of from about 100 to about 15,000, preferably from about 100 to 1000. Preferred emollients for use herein are isohexadecane, isononyl isononanoate, methyl isostearate, isopropyl isostearate, and mixtures thereof. A further emollient suitable for use in the composition of the present invention is petrolatum. The emollient material is preferably present in the compositions at a level of from about 0.1 % to about 10%.

The present compositions herein may comprise an emulsifier and/or surfactant, generally to help disperse and suspend the discontinuous phase within the continuous phase. For convenience hereinafter emulsifiers will be referred to under the term 'surfactants', thus 'surfactant(s)' will be used to refer to surface active agents whether used as emulsifiers or for other surfactant purposes such as skin cleansing. Known or conventional surfactants can be used in the composition, provided that the selected agent is chemically and physically compatible with essential components of the composition, and provides the desired characteristics. The compositions of the present invention preferably comprise from about 0.05% to about 10% of a surfactant or mixture of surfactants. The exact surfactant or surfactant mixture chosen will depend upon the pH of the composition and the other components present. The compositions of the present invention may comprise non-ionic, anioinic, cationic, zwitterionic, amphoteric surfactants, or mixtures thereof, preferably non-ionic.

Suitable nonionic surfactants useful herein are those that can be broadly defined as condensation products of long chain alcohols, e.g. C₈₋₃₀ alcohols, with sugar or starch polymers, i.e., glycosides, condensation products of alkylene oxides with fatty acids (i.e. alkylene oxide esters or diesters of fatty acids), condensation products of alkylene oxides with fatty alcohols (i.e. alkylene oxide ethers of fatty alcohols), condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol], polyhydroxy fatty acid amide surfactants, which are described in more detail in WO98/04241, sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of C₁-C₃₀ fatty alcohols, alkoxylated ethers of C1-C30 fatty alcohols, polyglyceryl esters of C₁-C₃₀ fatty acids, C₁-C₃₀ esters of polyols, C₁-C₃₀ ethers of polyols, alkyl phosphates, polyoxyalkylene fatty ether phosphates, fatty acid amides, acyl lactylates, and mixtures thereof. Preferred among the nonionic surfactants are those selected from the group consisting of cetearyl glucosides, cetearyl alcohols, PEG-100 stearate, sorbitan stearate and mixtures thereof.

Emulsions of the present invention may include a silicone containing emulsifier or surfactant. Useful silicone emulsifiers include dimethicone copolyols. Other examples include alkyl-modified dimethicone copolyols, i.e., compounds that contain C₂-C₃₀ pendant side chains. Still other useful dimethicone copolyols include materials having various cationic, anionic, amphoteric, and zwitterionic pendant moieties.

The compositions of the present invention may comprise a variety of other ingredients that are conventionally used in given product types provided that they do not unacceptably alter the benefits of the invention.

The optional components, when incorporated into the composition, should be suitable for use in contact with human keratinous tissue without undue toxicity, incompatibility, instability, allergic response, and the like within the scope of sound judgment. The *CTFA Cosmetic Ingredient Handbook,* Second Edition (1992) describes a wide variety of nonlimiting cosmetic and pharmaceutical ingredients commonly used in the skin care industry, which are suitable for use in the compositions of the present invention.

Certain embodiments of the present invention preferably contain a depilatory. As used herein, "depilatory" means an agent capable of removang hair from the skin by cleaving the disulfide bonds in hair keratin, thereby causing the hair fiber to disintegrate. Preferred depilatories useful in the subject invention include ammonium thioglycolate, barium sulfate, calcium thioglycolate, ethanolamine thioglycolate, potassium thioglycolate, sodium thioglycolate, thioglycolic acid and thioacetic acid. When present in the composition, the composition contains from about 0.0001_% to about 99.9_%, prefereably from about 0.001% to about 10%, and more preferably from about 0.01% to about 5%, by weight of the composition, of the depilatory. Examples of suitable depilatories are described in further detail in U. S. Patent No. 5,897,857.

The compositions of the present invention may further comprise a thickening agent. The cosmetic compositions of the present invention comprise from about 0.1% to about 5%, preferably from about 0.1% to about 3%, and more preferably from about 0.25% to about 2%, thickening agent by weight of the composition.

Suitable thickening agents include carboxylic acid polymers, crosslinked polyacrylates, polyacrylamides, xanthan gum and mixtures thereof, more preferably selected polyacrylamide polymers, xanthan gum and mixtures thereof. Preferred polyacrylamides include the non-ionic polymer under the CTFA designation: polyacrylamide and isoparaffin and laureth-7, available under the trade name Sepigel 305 from Seppic Corporation. More preferred for use herein are the co-polymer compositions commercially available from BASF Corp. under the tradename Luvigel EM™, the co-polymer compositions commercially available from Noveon as Novamer EC-1™ and the co-polymer compositions available from CIBA Speciality Chemicals, Macclesfield, UK, under the tradename Salcare SC91™.

The compositions of the present invention are useful for regulating keratinous tissue, particularly hair growth and mammalian skin condition. Such regulation of keratinous tissue conditions can include cosmetic prophylactic and therapeutic regulation. It may also include providing a more noticeable improvement, both tactile and visual, in the appearance and feel of the hair on the kin of a mammal. For example, such regulating methods are directed to making the hair appear softer, finer, and/or less noticeable. Also, such methods provide ease, frequency, and effectiveness of shaving on a mammal. Examples of regulating skin conditions include, but are not limited to softening and/or smoothing hair of a mammal, regulating skin texture (e.g. wrinkles and fine lines), increasing the rate of skin turnover, and improving skin color (e.g. redness, freckles).

Regulating keratinous tissue condition involves topically applying to the keratinous tissue a safe and effective amount of a composition of the present invention. In a preferred embodiment, the composition is chronically applied to the skin. By "chronic topical application" is meant continued topical application of the composition over an extended period during the subject's lifetime, preferably for a period of at least about one week, more preferably for a period of at least about one month, even more preferably for at least about three months, even more preferably for at least about six months, and more preferably still for at least about one year. While benefits are obtainable after various maximum periods of use (e.g., five, ten or twenty years), it is preferred that chronic application continue throughout the subject's lifetime. Typically applications would be on the order of about once per day over such extended periods, however application rates can vary from about once per week up to about three times per day or more.

A wide range of quantities of the compositions of the present invention can be employed to provide a skin appearance and/or feel benefit. Quantities of the present compositions, which are typically applied per application, are, in mg composition/cm² skin, from about 0.1 mg/cm² to about 20 mg/cm². A particularly useful application amount is about 0.5 mg/cm² to about 10 mg/cm².

Regulating keratinous tissue condition is preferably practiced by applying a composition which is intended to be left on the keratin structure for some esthetic, prophylactic, therapeutic or other benefit (i.e., a "leave-on" composition). After applying the composition to the skin, it is preferably left on the skin for a period of at least about 15 minutes, more preferably at least about 30 minutes, even more preferably at least about 1 hour, still more preferably for at least several hours, e.g., up to about 12 hours.

Another approach to ensure a continuous exposure of the skin to at least a minimum level of the skin care active is to apply the compound by use of a patch applied, e.g., to the face. The patch can be occlusive, semi-occlusive or non-occlusive and can be adhesive or non-adhesive. The composition can be contained within the patch or be applied to the skin prior to application of the patch. The patch can also include additional actives such as chemical initiators for exothermic reactions such as those described in U.S. Patents numbered 5,821,250, 5,981,547, and 5,972,957 to Wu, et al. The patch is preferably left on the skin for a period of at least about 5 minutes, more preferably at least about 15 minutes, more preferably still at least about 30 minutes, even more preferably at least about 1 hour, still more preferably at night as a form of night therapy.

The compositions of the present invention are manufactured by a process comprising the steps of mixing the water and alkane polyol having a ClogP of from +0.2 to +1.55, adding the amidine to the aqueous phase, and then heating the resulting mixture with mixing to a temperature of from 50°C to 95°C until the amidine dissolves. Following dissolution of the amidine in the aqueous phase, the premix solution is cooled and additional ingredients such as thickeners, silicones, additional actives and optional actives are added. It is necessary to form the amidine premix in the order above, because if the amidine is added to the water and subsequently heated without the alkane polyol having a ClogP of from +0.2 to +1.55, when the aqueous premix is cooled following dissolution of the amidine, the premix becomes supersaturated, and the amidine crystallises out of the aqueous premix. Furthermore, the amidine cannot be directly added to the alkane polyol, as it is not soluble in these materials, and the addition of water is required.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention.

### Examples I to V

| **INGREDIENTS** | **I** **% w/w** | **II** **% w/w** | **III** **% w/w** | **IV** **% w/w** | **V** **% w/w** |
|---|---|---|---|---|---|
| DEIONISED WATER | QS | QS | QS | QS | QS |
| DISODIUM EDTA | 0.1 | 0.1 | 0.1 | 0.1 | 0.1 |
| GLYCERIN | 10.0 | 15.0 | 10.0 | 25.0 | 10.0 |
| NIACINAMIDE | 3.5 | 3.50 | 5.0 | 5.0 | 3.5 |
| PANTHENOL | 0.5 | 1.0 | 1.0 | 1.0 | 0.5 |
| EMULGADE¹ | 0.2 | 0.2 | 0.2 | 0.2 | 0.2 |
| ISOHEXADECANE | 3.0 | 6.0 | 6.0 | 2.0 | -------- |
| ETHYL PARABEN | 0.15 | 0.15 | 0.15 | 0.15 | 0.15 |
| COCONUT OIL FRACTIONATED | -------- | ---------- | 0.2 | 3.0 | --------- |
| PROPYL PARABEN | 0.07 | 0.07 | 0.07 | 0.07 | 0.07 |
| STEARIC ACID | 0.1 | 0.1 | 0.1 | 0.1 | 0.1 |
| PEG-100 STEARATE² | 0.1 | 0.1 | 0.1 | 0.1 | 0.1 |
| STEARYL ALCOHOL | 0.61 | 0.61 | 0.61 | 0.61 | 0.61 |
| CETYL ALCOHOL | 0.49 | 0.49 | 0.49 | 0.49 | 0.49 |
| BEHENYL ALCOHOL | 0.40 | 0.40 | 0.40 | 0.40 | 0.40 |
| ISOPROPYL ISOSTEARATE | 1.5 | 3.0 | 1.5 | 1.5 | 0.65 |
| DL- α TOCOPHEROL ACETATE | 0.25 | 0.5 | 0.5 | 0.5 | 0.25 |
| MINERAL OIL | -------- | 1.0 | ----------- | ----------- | ----------- |
| PETROLATUM | 2.0 | -------- | 1.5 | 1.5 | 0.5 |
| LUVIGEL EM³ | -------- | -------- | 2.0 | 2.0 | 2.0 |
| SEPIGEL 305⁴ | 1.50 | 1.50 0 | ----------- | ----------- | ----------- |
| HEXAMIDINE DIISETHIONATE | 0.3 | 0.1 | 1.0 | --- | 0.3 |
| BENZAMIDINE | ----- | ------ | ------ | 0.5 | |
| SODIUM HYDROXIDE | 0.011 | 0.011 | 0.011 | 0.011 | ----------- |
| NYLONPOLY WL 10⁵ | 1.0 | 1.0 | 1.0 | 1.0 | 1.0 |
| DRY FLO PLUS⁶ | 1.0 | 1.5 | 2.0 | 2.0 | 1.0 |
| BENZYL ALCOHOL | 0.25 | 0.25 | 0.25 | 0.25 | 0.25 |
| DC 1503⁷ | 1.0 | 1.5 | 1.5 | 1.5 | 2.0 |
| PERFUME | 0.3 | 0.3 | 0.3 | 0.3 | 0.3 |
| OCTOCRYLENE | ----------- | ------------- | ----------- | ------------- | 1.0 |
| 1,2 HEXANEDIOL | 3.0 | 5.0 | --------- | ---------- | 3.0 |
| 1,2 OCTANEDIOL | ----------- | ------------ | 4.0 | ----------- | -------- |
| 1,7 HEPTANEDIOL | 0.2 | 0.4 | 0.2 | 0.4 | --------- |
| AVOBENZONE | ----------- | ------------- | ----------- | ------------- | 1.50 |
| OCTYL SALICYLATE | ----------- | ------------- | ----------- | ------------- | 3.5 |
| PHENYL BENZIMIDAZOLE SULPHONIC ACID | ----------- | ------------- | ----------- | ------------- | 1.0 |
| TRIETHANOL-AMINE | ----------- | ------------- | ----------- | ------------- | 0.61 |

| | | | | | |
|---|---|---|---|---|---|
| 1. Emulgade :Supplier : Cognis Deutchland GmbH, Germany. 2. PEG 100 Stearate supplied by ICI, England. 3. Luvigel EM:Supplier: BASF Plc, UK. 4. Sepigel 305: Supplier: Seppic, 75 Quai D'Orsay, Paris 5. Nylonpoly: Supplier Optima Chemicals, UK 6. Dry Flo Supplier: National Starch Chemical Company, USA 7. DC 1503: Supplied by Dow Coming, UK | | | | | |

The compositions are made as follows:

A water phase premix is prepared firstly by adding the polyol to water and then adding the amidine and then by admixing all water soluble ingredients, except sodium hydroxide and panthenol & Niacinamide and heating to about 75°C. A second premix is prepared by admixing of the oil soluble ingredients except the silicone oil (DC1503) and heating also to around 75°C. The oil phase is added to the water phase and sheared to form an emulsion. The emulsion is cooled to 60°C and the polymeric thickener added. At 55°C, sodium hydroxide solution is then added to neutralise to pH 6-7.5, except for examples where sunscreens are included. At 45-50°C the panthenol, benzyl alcohol, DC1503, dyes and particles are added and the resulting product is sheared to ensure particle dispersion, de-agglomeration and homogeneity. The composition can then be cooled to 40°C and perfume can be added. The product can then be prepared for packaging.

A moisturizing skin cream/lotion for hand and body skin care is prepared by conventional methods from the following components.

### Example VI

| **Component** | Ex. VI % w/w |
|---|---|
| Tospearl 144A | 1.000 |
| Sodium hydroxide 40% solution | 0.022 |
| Dimethicone and dimithiconol (DC 1503) | 1.000 |
| benzyl alcohol | 0.250 |
| D-panthenol | 1.000 |
| polyacrylamide (Sepigel 305) | 1.500 |
| coconut oil fractionated | 0.400 |
| DL-alpha tocopherol acetate | 0.250 |
| petrolatum (white soft paraffin) | 2.000 |
| isopropyl isostearate | 1.500 |
| Behynyl alcohol (Stenol 1822A) | 0.420 |
| cetyl alcohol 95% | 0.515 |
| stearic acid | 0.100 |
| stearyl alcohol (Crodacol S 95) | 0.641 |
| PEG-100-stearate (Myrj 59) | 0.100 |
| butylated hydroxytoluene | 1.000 |
| hexamidine diisethionate | 0.100 |
| ethyl paraben (Nipasol M) | 0.070 |
| isohexadecane (Arlamol HD) | 3.000 |
| Emulgade PL 68/50 | 0.200 |
| Glycerine | 7.000 |
| 1,2-hexanediol | 3.000 |
| Disodium EDTA | 0.100 |
| Deionised water, fragrance, presevatives | to 100% |

## Claims

1. A composition comprising:
a) an amidine;
b) an alkane polyol having a ClogP value of from +0.2 to +1.55, and
c) water.

2. The composition of claim 1 wherein the amidine comprises hexamidine, pentamidine, benzamidine or mixtures thereof.

3. The composition of claim 2 wherein the amidine comprises hexamidine.

4. The composition according to any of the preceding claims wherein the composition comprises 0.001% to 10% of said amidine.

5. The composition according to any of the preceding claims wherein the alkane polyol comprises a C₆ to C₉ polyol, or mixtures thereof, preferably a C₆ to C₉ diol, or mixtures thereof.

6. The composition of claim 5 wherein the alkane polyol comprises 1,2-hexanediol, 2-ethyl-1,3-hexanediol, 1,2-heptanediol, 1,7-heptanediol, 1,2-octanediol, 1,8-octanediol, 1,9-nonanediol, or mixtures thereof.

7. The composition of claim 6 wherein the alkane polyol comprises 1,2-hexanediol.

8. The composition according to claim 1 comprising from 0.01% to 20% of the alkane polyol.

9. The composition according to any of the preceding claims further comprising an additional skin care active.

10. The composition according to claim 9 wherein the additional skin care active comprises BHT or BHA, cetyl pyridinium chloride, green tea catechins, phytosterols, their salts and derivatives, vitamin B₃ compound, panthenol or its derivatives, retinoids, natural anti-inflammatory agents, antimicrobial actives, antifungal actives, sunscreen actives or mixtures thereof.

11. The composition of Claim 10 wherein the additional skin care active comprises BHT or BHA, vitamin B₃ compound, panthenol or its derivatives, or mixtures thereof, preferably panthenol or its derivatives.

12. The composition of Claim 11 wherein the additional skin care active comprises BHT.

13. The composition according to any of the preceding claims further comprising a humectant, preferably glycerine.

14. The composition according to any of the preceding claims further comprising a depilatory.

15. The composition according to any of the preceding claims having a pH of from about 4 to about 7.5.

16. A hair growth inhibiting composition comprising a composition according to any of the preceding claims.

17. The composition according to any of the preceding claims, wherein the composition is in the form of a water-in-silicone emulsion.

18. The composition according to any of the preceding claims further comprising a surfactant.

19. A method of inhibiting mammalian hair growth, said method comprising the step of topically applying a safe and effective amount of the composition according to claim 1 to the skin of a mammal.

20. The method of claim 19, wherein said method comprises the step of applying from about 0.1 mg/cm² to 20 mg/cm² of the composition according to any of the preceding claims.

21. Non therapeutic use of alkane polyols having a ClogP of from +0.2 to +1.55 in a composition comprising a hair growth inhibitor for modulating the delivery and efficacy of said hair growth inhibitor on the skin wherein said hair growth inhibitor is an amidine.

22. Process of making a composition according to claims 1 to 18 comprising the steps of:
a) forming a premix of water and an alkane polyol having a ClogP of from +0.2 to +1.55;
b) adding an amidine to the premix and heating with mixing to dissolve said amidine to form an amidine solution; and
c) cooling said amidine solution.

23. The process according to claim 24, wherein said premix plus amidine is heated to a temperature of from 50°C to 95°C.

24. A method of reducing shaving frequency, said method comprising the step of topically applying a safe and effective amount of the composition according to any of the preceding claims to the skin of a mammal.

## Patentansprüche

1. Zusammensetzung, umfassend:
a) ein Amidin;
b) ein Alkanpolyol mit einem ClogP-Wert von +0,2 bis +1,55 und
c) Wasser.

2. Zusammensetzung nach Anspruch 1, wobei das Amidin Hexamidin, Pentamidin, Benzamidin oder Mischungen davon umfasst.

3. Zusammensetzung nach Anspruch 2, wobei das Amidin Hexamidin umfasst.

4. Zusammensetzung nach einem der vorstehenden Ansprüche, wobei die Zusammensetzung zu 0,001 % bis 10 % das Amidin umfasst.

5. Zusammensetzung nach einem der vorstehenden Ansprüche, wobei das Alkanpolyol ein C₆- bis C₉-Polyol oder Mischungen davon umfasst, vorzugsweise ein C₆- bis C₉-Diol oder Mischungen davon.

6. Zusammensetzung nach Anspruch 5, wobei das Alkanpolyol 1,2-Hexandiol, 2-Ethyl-1,3-hexandiol, 1,2-Heptandiol, 1,7-Heptandiol, 1,2-Octandiol, 1,8-Octandiol, 1,9-Nonandiol oder Mischungen davon umfasst.

7. Zusammensetzung nach Anspruch 6, wobei das Alkanpolyol 1,2-Hexandiol umfasst.

8. Zusammensetzung nach Anspruch 1, die zu 0,01 % bis 20 % das Alkanpolyol umfasst.

9. Zusammensetzung nach einem der vorstehenden Ansprüche, die ferner einen zusätzlichen Hautpflegewirkstoff umfasst.

10. Zusammensetzung nach Anspruch 9, wobei der zusätzliche Hautpflegewirkstoff BHT oder BHA, Cetylpyridiniumchlorid, Grünteecatechine, Phytosterole, deren Salze und Derivate, Vitamin-B₃-Verbindung, Panthenol oder dessen Derivate, Retinoide, natürliche entzündungshemmende Mittel, antimikrobielle Wirkstoffe, pilzbefallverhütende Wirkstoffe, Sonnenschutzmittel oder Mischungen davon umfasst.

11. Zusammensetzung nach Anspruch 10, wobei der zusätzliche Hautpflegewirkstoff BHT oder BHA, Vitamin-B₃-Verbindung, Panthenol oder dessen Derivate oder Mischungen davon umfasst, vorzugsweise Panthenol oder dessen Derivate.

12. Zusammensetzung nach Anspruch 11, wobei der zusätzliche Hautpflegewirkstoff BHT umfasst.

13. Zusammensetzung nach einem der vorstehenden Ansprüche, die ferner ein Feuchthaltemittel, vorzugsweise Glycerin, umfasst.

14. Zusammensetzung nach einem der vorstehenden Ansprüche, die ferner ein Enthaarungsmittel umfasst.

15. Zusammensetzung nach einem der vorstehenden Ansprüche mit einem pH von ungefähr 4 bis ungefähr 7,5.

16. Den Haarwuchs hemmende Zusammensetzung, umfassend eine Zusammensetzung nach einem der vorstehenden Ansprüche.

17. Zusammensetzung nach einem der vorstehenden Ansprüche, wobei die Zusammensetzung in der Form einer Wasser-in-Silikon-Emulsion vorliegt.

18. Zusammensetzung nach einem der vorstehenden Ansprüche, die ferner ein Tensid umfasst.

19. Verfahren zum Hemmen des Haarwuchses bei Säugern, wobei das Verfahren den Schritt des örtlichen Auftragens einer sicheren und wirksamen Menge der Zusammensetzung nach Anspruch 1 auf die Haut eines Säugers umfasst.

20. Verfahren nach Anspruch 19, wobei das Verfahren den Schritt des Auftragens von ungefähr 0,1 mg/cm² bis 20 mg/cm² der Zusammensetzung nach einem der vorstehenden Ansprüche umfasst.

21. Nichttherapeutische Verwendung von Alkanpolyolen mit einem ClogP von +0,2 bis +1,55 in einer Zusammensetzung, die einen Haarwuchshemmstoff umfasst, zum Anpassen der Abgabe und Wirksamkeit des Haarwuchshemmstoffes auf der Haut, wobei der Haarwuchshemmstoff ein Amidin ist.

22. Verfahren zur Herstellung einer Zusammensetzung nach Ansprüchen 1 bis 18, das die folgenden Schritte umfasst:
a) Bilden einer Vormischung aus Wasser und einem Alkanpolyol mit einem ClogP von +0,2 bis +1,55;
b) Hinzufügen eines Amidins zu der Vormischung und Erwärmen unter Mischen, um das Amidin aufzulösen, um eine Amidinlösung zu bilden; und
c) Abkühlen der Amidinlösung.

23. Verfahren nach Anspruch 24, wobei die Vormischung plus Amidin auf eine Temperatur von 50 °C bis 95 °C erwärmt wird.

24. Verfahren zum Verringern der Rasierhäufigkeit, wobei das Verfahren den Schritt des örtlichen Auftragens einer sicheren und wirksamen Menge der Zusammensetzung nach einem der vorstehenden Ansprüche auf die Haut eines Säugers umfasst.

## Revendications

1. Composition comprenant :
a) une amidine ;
b) un polyol alcane ayant une valeur de ClogP comprise entre +0,2 et +1,55, et
c) de l'eau.

2. Composition selon la revendication 1, dans laquelle l'amidine comprend de l'hexamidine, de la pentamidine, de la benzamidine ou leurs mélanges.

3. Composition selon la revendication 2, dans laquelle l'amidine comprend de l'hexamidine.

4. Composition selon l'une quelconque des revendications précédentes, dans laquelle la composition comprend de 0,001 % à 10 % de ladite amidine.

5. Composition selon l'une quelconque des revendications précédentes, dans laquelle le polyol alcane comprend un polyol en C₆ à C₉, ou leurs mélanges, de préférence un diol en C₆ à C₉, ou leurs mélanges.

6. Composition selon la revendication 5, dans laquelle le polyol alcane comprend du 1,2-hexanediol, du 2-éthyle-1,3-hexanediol, du 1,2-heptanediol, du 1,7-heptanediol, du 1,2-octanediol, du 1,8-octanediol, du 1,9-nonanediol, ou leurs mélanges.

7. Composition selon la revendication 6, dans laquelle le polyol alcane comprend du 1,2-hexanediol. 1.

8. Composition selon la revendication 1, comprenant de 0,01 % à 20 % de polyol alcane.

9. Composition selon l'une quelconque des revendications précédentes, comprenant, en outre, un agent actif de soin de la peau supplémentaire.

10. Composition selon la revendication 9, dans laquelle l'agent actif de soin de la peau supplémentaire comprend du BHT ou du BHA, du chlorure de cétyl-pyridinium, des catéchines de thé vert, des phytostérols, leurs sels et dérivés, un composé de vitamine B₃, du panthénol ou ses dérivés, des rétinoïdes, des agents naturels anti-inflammatoires, des agents actifs antimicrobiens, des agents actifs antifongiques, des agents actifs contre les rayons solaires ou leurs mélanges.

11. Composition selon la revendication 10, dans laquelle l'agent actif de soin de la peau supplémentaire comprend du BHT ou du BHA, un composé de vitamine B3, du panthénol ou ses dérivés, ou leurs mélanges, de préférence du panthénol ou ses dérivés.

12. Composition selon la revendication 11, dans laquelle l'agent actif de soin de la peau supplémentaire comprend du BHT.

13. Composition selon l'une quelconque des revendications précédentes, comprenant, en outre, un humidifiant, de préférence de la glycérine.

14. Composition selon l'une quelconque des revendications précédentes, comprenant, en outre, un dépilatoire.

15. Composition selon l'une quelconque des revendications précédentes, ayant un pH compris entre environ 4 et environ 7,5.

16. Composition inhibant la pousse des cheveux comprenant une composition selon l'une quelconque des revendications précédentes.

17. Composition selon l'une quelconque des revendications précédentes, dans laquelle la composition est sous la forme d'une émulsion eau-dans-silicone.

18. Composition selon l'une quelconque des revendications précédentes, comprenant, en outre, un agent tensioactif.

19. Procédé d'inhibition de la pousse des cheveux mammalienne, ledit procédé comprenant l'étape d'application topique d'une quantité sûre et efficace de la composition selon la revendication 1, sur la peau d'un mammifère.

20. Procédé selon la revendication 19, dans lequel ledit procédé comprend l'étape d'application d'environ 0,1 mg/cm² à 20 mg/cm² de la composition selon l'une quelconque des revendications précédentes.

21. Utilisation non thérapeutique de polyols alcanes ayant une valeur de ClogP comprise entre +0,2 et +1,55 dans une composition comprenant un inhibiteur de la pousse des cheveux pour moduler la libération et l'efficacité dudit inhibiteur de la pousse des cheveux sur la peau, dans laquelle ledit inhibiteur de la pousse des cheveux est une amidine.

22. Procédé de fabrication d'une composition selon les revendications 1 à 18, comprenant les étapes consistant à :
a) former un prémélange d'eau et d'un polyol alcane ayant une valeur de ClogP comprise entre +0,2 et +1,55 ;
b) ajouter une amidine au prémélange et chauffer en mélangeant pour dissoudre ladite amidine pour former une solution d'amidine ; et
c) refroidir ladite solution d'amidine.

23. Procédé selon la revendication 24, dans lequel ledit prémélange plus l'amidine sont chauffés pour atteindre une température comprise entre 50 °C et 95 °C.

24. Procédé de réduction de la fréquence de rasage, ledit procédé comprenant l'étape d'application topique d'une quantité sûre et efficace de la composition selon l'une quelconque des revendications précédentes, sur la peau d'un mammifère.
